# EUROPEAN PATENT APPLICATION

(11) **EP 3 048 443 A1**
(43) Date of publication of application: **27.07.2016**
(21) Application number: 16152333.7
(22) Date of filing: 22.01.2016
(51) Int. Cl.: G01N 27/417

(54) **MULTISENSING MULTIPARAMETER DESIGN USING DYNAMIC PARALLEL RESISTANCES ON SENSING ELEMENT SUBSTRATE**

(30) Priority: 22.01.2015 US 201514602344
(71) Applicant: Delphi Technologies, Inc., Troy, MI 48007 (US)
(72) Inventor: COVARRUBIAS, Alfredo Ibarra, Oxford, Michigan 48371 (US); KULKARNI, Vishal, Lake Orion, Michigan 48360 (US)
(74) Representative: Delphi France SAS

(57) **Abstract**

An exhaust sensor (10) comprises a temperature cell (34/38/36) and a calibration resistor (94). The temperature cell (34/38/36) has a characteristic such that a temperature cell impedance varies as a function of the exhaust sensor temperature, with the temperature cell (34/38/36) having a high impedance at ambient temperature and a relatively low impedance at the operating temperature of the exhaust sensor (10), the operating temperature being higher than ambient temperature. The calibration resistor (94) is electrically connected in parallel with the temperature cell (34/38/36). A method of producing and a method of using such an exhaust sensor (10) are also disclosed.

## Description

### BACKGROUND OF THE INVENTION

Exhaust sensors are commonly used in a vehicle emission control system to measure a constituent of the exhaust gas of the vehicle. To compensate for part-to-part variability between sensors, some sensor assemblies include a calibration resistor, typically located in the sensor connector or wiring harness. This calibration resistor is calibrated to a specific resistance value based on a performance characteristic of the sensor measured during construction of the sensor assembly. In use, a controller connected to the sensor assembly reads the resistance value of the calibration resistor. Based on the resistance value of the calibration resistor, the controller can compensate for the actual performance of the specific sensor assembly.

Including a calibration resistor in the connector assembly or wiring harness has certain associated disadvantages. Extra wires may be required in the wiring harness to provide the resistance signal to the controller. For example, a sensor assembly having six connections may require an eight-pin connector to accommodate an additional two wires from the calibration resistor to the controller. The sensor manufacturing process may additionally be complicated by the need to keep track of a particular sensor and a particular calibration resistor until such time as the sensor / connector / wiring harness assembly is complete, or by the need to fixture, laser trim, and passivate a resistor incorporated in a connector or wiring harness.

Accordingly, improvements are always sought in the art.

### BRIEF SUMMARY OF THE INVENTION

In a first aspect of the invention, an exhaust sensor comprises a temperature cell and a calibration resistor. The temperature cell has a characteristic such that a temperature cell impedance varies as a function of the exhaust sensor temperature, with the temperature cell having a high impedance at ambient temperature and a relatively low impedance at the operating temperature of the exhaust sensor, the operating temperature being higher than ambient temperature. The calibration resistor is electrically connected in parallel with the temperature cell.

In a second aspect of the invention, a method of producing an exhaust sensor includes the steps of forming a temperature cell having an impedance that varies as a function of temperature, and disposing a calibration resistor electrically in parallel with the temperature cell.

In a third aspect of the invention, a method of using an exhaust sensor includes the steps of measuring the resistance of a calibration resistor that is electrically in parallel with a temperature cell at a time when the exhaust sensor is cool, that is at or near ambient temperature, and using the measured resistance value in a controller to determine a compensation for a performance characteristic of the exhaust sensor.

More specifically, it is disclosed an exhaust sensor operable at an operating temperature greater than ambient temperature. The exhaust sensor comprises a temperature cell comprising an electrolyte material in electrical contact with a first electrode and a second electrode and having a characteristic such that temperature cell impedance measured between the first electrodes and, the second electrode varies as a function of the temperature of the electrolyte material, and a resistor having a fixed resistance value, said resistor electrically connected between the first electrode and the second electrode, the resistance value of the resistor being intermediate between the temperature cell impedance when the exhaust sensor is at ambient temperature and the temperature cell impedance when the exhaust sensor is at the operating temperature.

More particularly, the fixed resistance value is selected as a function of a performance characteristic of the exhaust sensor.

Also, the performance characteristic of the exhaust sensor is a sensitivity of the exhaust sensor to a concentration of a gas species.

Also, the resistor is formed as a film resistor deposited on a substrate.

Also, the resistor is trimmed to the fixed resistance value using laser trimming.

The invention also covers a method of producing an exhaust sensor, said method comprising the steps of
disposing a first electrode and a second electrode in contact with an electrolyte to form a temperature cell;
disposing an resistor electrically connected to the first electrode and the second electrode;
determining a performance characteristic of the exhaust sensor ; and
adjusting the resistance value of the resistor based on the determined performance characteristic of the exhaust sensor.

More particularly, the performance characteristic of the exhaust sensor is a sensitivity of the exhaust sensor to a concentration of a gas species.

The invention also covers a method of using an exhaust sensor, said exhaust sensor comprising a fixed resistor electrically in parallel with a temperature cell hose impedance varies as a function of the temperature of the exhaust sensor. The method comprises the steps of:
disposing the exhaust sensor in an exhaust system so as to be exposed to exhaust gas;
measuring the impedance of the parallel combination of the fixed resistor and the temperature cell when the exhaust sensor is at ambient temperature;
providing the measured ambient temperature impedance value to a controller configured to use the ambient temperature impedance value to determine a sensor compensation characteristic;
receiving a concentration signal from the exhaust sensor, said concentration signal being a function of a gas species concentration sensed by the exhaust sensor; and
Adjusting the concentration signal based on the sensor compensation characteristic to determine a compensated gas species concentration.

More particularly, the exhaust sensor is disposed in a vehicle and wherein the step of measuring the impedance of the parallel combination of the fixed resistor and the temperature cell is performed at a key-on event of the vehicle.

### BRIEF DESCRIPTION OF THE DRAWINGS

Example sensors will now be described with reference to the accompanying drawings, which are meant to be illustrative, not limiting, and wherein like elements are numbered alike in several figures, in which:
Figure 1 is an expanded perspective view of one embodiment of a sensor element that includes a calibration resistor.
Figure 2 is a schematic elevational end view of the sensing end of the sensor element of Figure 1.
Figure 3 is a cross-sectional view of a sample gas sensor.
Figure 4 is a schematic block diagram of the sensor element of Figure 1 and a sensor circuit for use therewith.

### DETAILED DESCRIPTION OF THE INVENTION

The terms "first," "second," and the like, herein do not denote any order, quantity, or importance, but rather are used to distinguish one element from another, and the terms "a" and "an" herein do not denote a limitation of quantity, but rather denote the presence of at least one of the referenced item. Additionally, all ranges set forth herein are inclusive and combinable. For example, a range of up to about 500 micrometers (µm), e.g., a thickness of about 25 µm to about 500 µm; or a thickness of about 50 µm to about 200 µm, includes the ranges of about 25 µm to about 200 µm and about 50 µm to about 500 µm, without the need for explicit statement thereof. The modifier "about" used in connection with a quantity is inclusive of the stated value and has the meaning dictated by the context (e.g., includes the usual degree of error associated with measurement of the particular quantity).

A gas sensor and associated sensor circuitry for sensing ammonia is presented herein to provide description of aspects of the present invention. A representative ammonia sensor without a calibration resistor is described in U.S. Patent Application Publication No. 2006/0151338, the entire disclosure of which is incorporated herein by reference. It is to be understood that the present invention is not limited to use with ammonia sensing, but may be used with any sensor that would benefit from a calibration resistor.

The gas sensor includes a cell (i.e., electrodes disposed on opposite sides of an electrolyte for ionic communication from one electrode to the other via the electrolyte) capable of generating output signals responsive to ammonia (an "ammonia-sensing cell") in an unknown gas (i.e., a gas of unknown ammonia content) to which the cell is exposed. The gas sensor also includes a cell capable of generating an output signal responsive to the oxygen and/or hydrocarbon in the unknown gas (an "A/F cell"), from which signal the air/fuel ratio of the gas can be determined. A sensor circuit in communication with the ammonia-sensing cell and the A/F cell processes signals emitted by those cells to determine the ammonia content of the gas. Optionally, one or more of the sensing cells and/or pump cells, e.g., the ammonia-sensing cell and/or the A/F cell, can be formed as separate sensor elements. Alternatively, all of the sensing cells comprise part of the same, monolithic sensor element. A method for sensing ammonia in the unknown gas can be carried out by exposing the ammonia-sensing cell and the A/F cell to the unknown gas and employing the signal from the A/F cell to obtain data reflecting the oxygen and water vapor content of the gas, which data can be employed with the signal from the ammonia-sensing cell to determine the ammonia gas content of the gas.

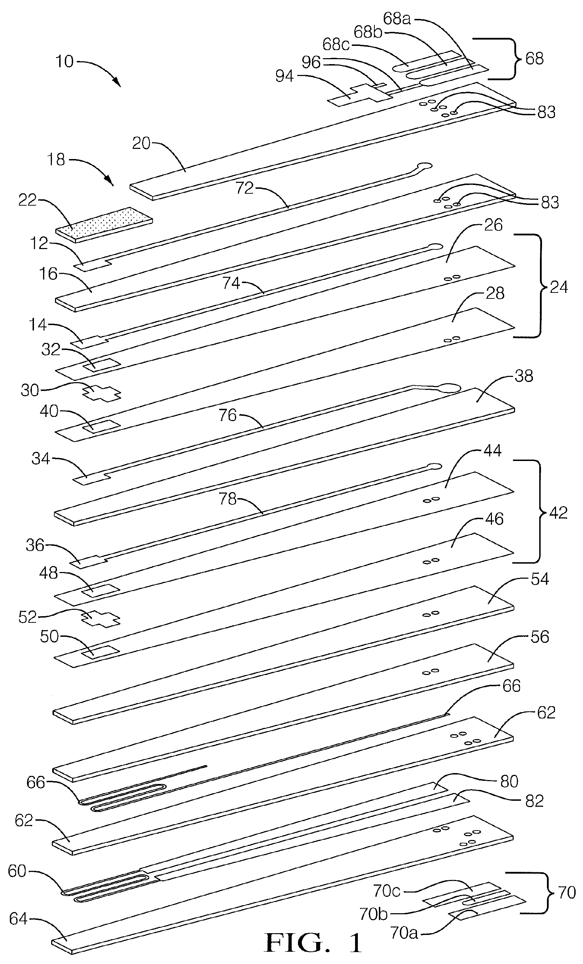

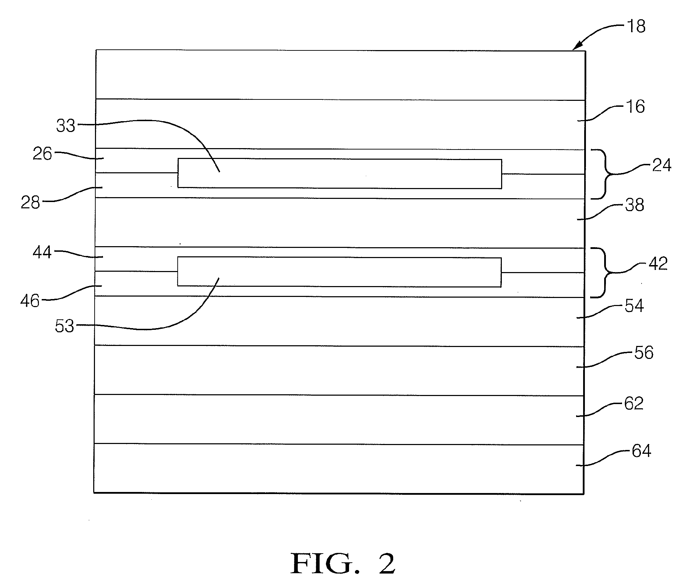

One embodiment of a sensor element for use with the described circuitry and method is illustrated in Figures 1 and 2. As described more fully below, sensor element 10 is a monolithic structure formed by the lamination of various layers that comprise an electrochemical ammonia-sensing cell (12/16/14), an electrochemical A/F cell and a heater, with first and second insulating layers between the electrochemical cells and the heater.

Electrochemical ammonia-sensing cell (12/16/14) comprises ammonia cell electrodes separated by an electrolyte for ionic communication therethrough. More specifically, ammonia-selective sensing electrode 12 and reference electrode 14 (the ammonic cell electrodes) are disposed on opposite sides of a solid electrolyte layer 16 (the ammonia cell electrolyte). On the side of sensing electrode 12 opposite solid electrolyte layer 16 is a protective layer 18, which optionally comprises a dense section 20 and a porous section 22 that enables fluid communication between sensing electrode 12 and the unknown gas (i.e., section 22 protects the electrode 12 from abrasion and/or poisoning while permitting the unknown gas to contact electrode 12).

Protective layer 18 is designed to protect the electrode 12 from contaminants, to provide structural integrity to the sensor element 10 and the electrode 12, and to allow the electrode 12 to sense ammonia gas without inhibiting the performance of the sensor element 10.

The material forming sensing electrode 12 is reactive with ammonia in the unknown gas, and exposure of electrode 12 to ammonia in the unknown gas elicits an electrochemical reaction at electrode 12. The sensing electrode 12 can comprise any ammonia-selective material compatible with the operating environment in which sensor element 10 will be used. Ammonia-selective materials comprise a primary material and a dopant secondary material.

An electrode connecting material is combined with a portion of the ammonia-selective material of sensing electrode 12 to facilitate the establishment of electrical continuity between the ammonia-selective material and a lead conductor such as lead 72. The electrode connecting material can comprise an electrically conductive metal and/or conductive metal oxide material with which a lead wire can easily make an electrical connection. These electrode connecting materials can form composites with the ammonia-selective material by mutual intermixing or by simple physical contact with each other (by thin-film or thick-film deposition means) and thus enable the ammonia-selective material to make an electrical connection to a lead wire adequate to enable the measurement of an emf signal from the ammonia-sensing cell 12/16/14 to be made via the lead wire. To avoid affecting the emf generated by the ammonia-selective material of the sensing electrode 12, the electrode connecting material does not contact both the electrolyte layer 16 and the unknown gas. Therefore, the electrode connecting material, if it is in contact with the electrolyte layer 16, can be covered by an insulating layer (like top layer 20) to shield it from the unknown gas or, before the electrode connecting material is applied, an insulating layer (not shown) can be applied to the electrolyte where needed to prevent contact between the electrode connecting material and the electrolyte 16, in which case there is no need to shield the electrode connecting material from the unknown gas.

The ammonia-selective material for the sensing electrode 12 can be formed in advance of deposition onto the electrolyte layer 16 or can be disposed on the electrolyte layer 16 and formed during the firing of the sensor element.

In one embodiment, the ammonia-selective material is prepared and is disposed onto the electrolyte (or the layer adjacent the electrolyte). In this method, the primary material, preferably in the form of an oxide, is combined with the dopant secondary material and optional other dopants, if any, simultaneously or sequentially. By either method, the materials are preferably well-mixed to enable the desire incorporation of the dopant secondary material and any optional dopants into the primary material to produce the desired ammonia-selective material. Once the ammonia-selective material has been prepared, it can be made into an ink and disposed onto the desired sensor layer.

If an ink is employed, beside the above metals/oxides/dopants, it can also comprise binder(s), carrier(s), wetting agent(s), and the like, and combinations comprising at least one of the foregoing. The binder can be any material capable of providing adhesion between the ink and the substrate.

In contrast to the sensing electrode 12, the reference electrode 14 can comprise any electrode material, i.e., it does not need to be sensitive to NH₃. The reference electrode 14 can comprise any catalyst capable of producing an electromotive force across the electrolyte layer 16 when the sensing electrode 12 contacts NH₃, including metals such as platinum, palladium, gold, osmium, rhodium, iridium, ruthenium, - and the like, as well as alloys, and combinations comprising at least one of the foregoing catalysts

Fugitive materials, i.e., materials that degrade and leave voids in the electrode upon firing, can be employed in the electrode formulations to provide porosity to electrodes, e.g., a porosity sufficient to enable the ammonia to enter the electrode and reach triple points (points where the electrode, electrolyte, and ammonia meet to enable the desired reactions).

With respect to the size and geometry of the sensing and reference electrodes 12, 14, they are generally adequate to provide current output sufficient to enable reasonable emf signal resolution over a wide range of ammonia concentrations. Generally, a thickness of about 1.0 micrometers to about 25 micrometers can be employed, for example, about 5 micrometers to about 20 micrometers, optionally about 10 micrometers to about 18 micrometers. The geometry of the electrodes can be substantially similar to the geometry of the electrolyte.

Electrodes can be formed using techniques such as chemical vapor deposition, screen printing, sputtering, and stenciling, with screen printing the sensing and reference electrodes onto appropriate tapes being preferred due to simplicity, economy, and compatibility with the subsequent firing process. For example, reference electrode 14 can be screen printed onto support layer 24 or over the electrolyte layer 16, and the sensing electrode 12 can be screen printed under porous protective layer 18 or over the electrolyte layer 16.

Electrolyte layer 16, like other electrolyte layers referred to herein, can comprise any material that is compatible with the environment in which the gas sensor will be utilized (e.g., up to about 1,000°C) and is capable of permitting the electrochemical transfer therethrough of ions generated at one of the electrodes 12 and 14 to the other while inhibiting the physical passage of the unknown gas therethrough. Possible electrolyte materials can comprise metal oxides such as zirconia, and the like, which can optionally be stabilized or partially stabilized with calcium, barium, yttrium, magnesium, aluminum, lanthanum, cesium, gadolinium, and the like, and oxides thereof, as well as combinations comprising at least one of the foregoing electrolyte materials. For example, the electrolyte can be alumina and yttrium-stabilized zirconia. The electrolyte establishes ionic communication between the electrodes disposed on opposite sides thereof.

An electrolyte such as layer 16 with the electrodes 12 and 14 thereon can be formed via many processes (e.g., die pressing, roll compaction, stenciling and screen printing, tape casting techniques, and the like) and can have a thickness of up to about 500 micrometers (µm), e.g., a thickness of about 25 µm to about 500 µm; or a thickness of about 50 µm to about 200 µm.

Disposed on the side of ammonia-sensing cell 12/16/14 opposite insulating support layer 18 are insulating layer(s), e.g., bifurcated insulating support layer 24 comprising a first insulating support layer 26 and a second insulating support layer 28. An insulating layer such as insulating support layer 24 provides structural integrity (e.g., it enhances the physical strength of the sensor), and physically separates and electrically isolates components on either side thereof. For example, support layer 24 can electrically isolate an electrode, such as electrode 14, from another electrode, e.g., electrode 34. An insulating layer can comprise a dielectric material such as alumina (e.g., delta alumina, gamma alumina, theta alumina, and combinations comprising at least one of the foregoing aluminas), and the like.

Aperture 32 in layer 26, like other apertures and open channels described herein, can be formed by perforating or cutting the layer before the layer is incorporated into the sensor element. For manufacturing purposes, the aperture or channel can be filled with fugitive material (not shown) that is later burned away during the manufacture of the sensor element 10. The fugitive material can comprise, for example, carbon, graphite, an insoluble organic material, a polymeric material, or the like. Aperture 32 permits fluid communication of an unknown gas with electrode 14 via an open aperture 33 (Figure 2) between layer 26 and layer 28 that is open to the unknown gas. Like aperture 32, open aperture 33 is formed upon the removal of fugitive material 30 (Figure 1) which is deposited between layers 26 and 28 and is later burned away during the manufacture of sensor element 10. Aperture 32 and aperture 33 cooperate to form an aperture configured to permit fluid communication of an unknown gas with an ammonia cell electrode and with an A/F cell electrode, i.e., with the ammonia-sensing cell and with the A/F cell.

On a side of layer 24 opposite ammonia-sensing cell 12/16/14 is an A/F cell 34/38/36 comprising A/F cell electrodes separated by an electrolyte for ionic communication therethrough. More specifically, A/F cell 34/38/36 comprises pump electrodes 34 and 36 (the A/F cell electrodes) disposed on opposite sides of an electrolyte layer 38 (the A/F cell electrode). Electrodes 34 and 36 can comprise any material suitable for oxygen pump electrodes.

Electrolyte layer 38, like other electrolyte layers referred to herein, can comprise any material that is compatible with the environment in which the gas sensor will be utilized (e.g., up to about 1,000°C) and is capable of permitting the electrochemical transfer therethrough of ions generated at one of the electrodes 34 and 36 to the other while inhibiting the physical passage of the unknown gas therethrough. The electrolyte establishes ionic communication between the electrodes disposed on opposite sides thereof. Exemplary electrolyte materials include (but are not limited to) zirconia which can optionally be stabilized or partially stabilized with calcium, barium, yttrium, magnesium, aluminum, lanthanum, cesium, gadolinium, and the like, as well as combinations comprising at least one of the foregoing, any of which can be present in oxide form. In a particular illustrative embodiment, electrolyte layer 38 can comprise yttria-partially-stabilized zirconia.

Electrode 34 is in fluid communication with an aperture 40 in layer 28 and thus with the open aperture formed by the fugitive material 30, and thus with electrode 14.

On the side of A/F cell 34/38/36 opposite from ammonia-sensing cell 12/16/14 is an insulating support layer 42 which, in the illustrated embodiment, is bifurcated and comprises a first layer 44 and a second layer 46. First layer 44 has an aperture 48 and second layer 46 has an aperture 50. Apertures 48 and 50 cooperate to provide a gas diffusion chamber in layer 42. A porous material 52 between layer 44 and layer 46 provides a gas diffusion limiting aperture 53 (Figure 2) which limits gas flow between the unknown gas and apertures 48 and 50. Thus, apertures 48, 50 and 53 cooperate to form an aperture for fluid communication of the unknown gas with A/F cell 34/38/36. Porous material 52 can be formed, for example, from a deposit of a printable ink comprising a mixture of a particulate refractory oxide, e.g., alumina, and a fugitive material onto one of layers 44 and 46. During the manufacture of sensor element 10, the ink is exposed to an elevated temperature and the fugitive material is burned away, leaving a porous aperture of a corresponding shape and known porosity. Other diffusion-limiting apertures or channels disclosed herein can be formed in a similar manner. As a result of apertures 33 and 53, an unknown gas to which sensor element 10 is exposed can contact both A/F cell electrodes. When a constant potential is applied to electrodes 34 and 36, the current through A/F cell 34/38/36 (the A/F cell signal) is limited by the oxygen available via aperture 53 and reflects the partial pressure of oxygen in the unknown gas. Therefore, the A/F cell signal indicates the air-to-fuel ratio of the unknown gas.

On the side of insulating layer 46 opposite from A/F cell 34/38/36 can be an optional electrolyte layer 54, and on the side of layer 54 opposite from layer 42 are insulating layer(s) 56. A heater 60 is disposed on the side of insulating layer 56 opposite from layer 54, between insulating layers 62 and 64. Insulating layer 62 is adjacent insulating layer 56, but there is disposed between them an optional metallic electromagnetic barrier 66 on the side of layer 62 opposite from heater 60. Because heater 60 is part of the monolithic structure of sensor element 10, it is in thermal communication with A/F cell 34/38/36 and ammonia-sensing cell 12/16/14, i.e., heater 60 can be used for maintaining sensor element 10 and the cells therein at a selected working temperature. In other embodiments, a heater could be in thermal communication with the A/F cell and/or with the ammonia-sensing cell without necessarily being part of a monolithic laminate structure with them, e.g., simply by being in close physical proximity to a cell.

Contact pads 68a, 68b, 68c, 70a, 70b, and 70c comprise electrically conductive material and facilitate electrical communication between sensor element 10 and a sensor circuit that can include sources of current and electrical potential and circuitry responsive to the electrolytic cells in sensor element 10 to indicate the concentration of at least one gas species, as described herein. Several leads are provided in sensor element 10 to provide electrical communication between the control unit and the electrodes and heating member in sensor element 10. Lead 72 is in electrical communication with (i.e., is connected to) sensing electrode 12 and contact pad 68c. Lead 74 is in electrical communication with the reference electrode 14 and contact pad 68b. Similarly, lead 76 is in electrical communication with electrode 34 and contact pad 68a, and lead 78 is in electrical communication with electrode 36 and contact pad 70b. Leads 80 and 82 are in electrical communication with heater 60 and with contact pads 70a and 70c.

The various leads are in electrical communication with contact pads 68a, 68b, 68c, 70a, 70b, and 70c through vias such as vias 83 formed in layers 16, 20, 26, 28, 38, 44, 46, 54, 56, 62 and 64. The vias comprise electrically conductive materials and provide a medium for establishing electrical communication between the leads and the contact pads 68a, 68b, 68c, 70a, 70b, and 70c. A via can be formed by perforating the substrate to form a through- hole at a selected position, filling the through-hole with a conducting paste, and curing the conducting paste while the substrate is shaped and cured under heat in a heating/pressing step. The conducting paste can be prepared as a paste using conducting particles, a thermosetting resin solution, and, if necessary, a solvent. The thermosetting resin can be selected from resins that can be cured simultaneously in the step of heating/pressing the substrate. For example, an epoxy resin, thermosetting polybutadiene resin, phenol resin, and/or polyimide resin can be used.

For the conducting particles, a conducting particle-forming powder of a metal material that is stable and has a low specific resistance and low mutual contact resistance is preferably used. For example, a powder of gold, silver, copper, platinum, palladium, lead, tin, and/or nickel, or a combination comprising at least one of the foregoing can be used to form the vias. In one embodiment, the vias are formed at a position on sensor element 10 conveniently distanced from the electrodes, e.g., vias can be formed at one end of sensor element 10 and the opposite end of the sensor element 10 can be the sensing end or tip, at which the electrodes are disposed.

Sensor element 10 and contact pads 68a, 68b, 68c, 70a, 70b, and 70c can be configured to receive a wiring harness by which electrical communication can be established between sensor element 10 and a sensor circuit. Sensor element 10 can be manufactured using thick film, multi-layer technology including, e.g., the use of strips of commercially suitable alumina, zirconia, etc., for the electrolyte and insulating layers in which vias can be formed as needed and on which electrodes and fugitive compounds thereon can be printed using suitable ink compounds. Such printed tapes can be assembled and fired (e.g., co-fired) into a laminated monolithic (i.e., single structure) sensor element having electronic contact pads on opposite outside surfaces thereof. The disclosed sensor elements can also be built into monolithic structures by bulk ceramic technology, or thick-film multi-layer technology, or thin-film multi-layer technology. In bulk ceramic technology, the sensors are formed in a cup shape by traditional ceramic processing methods with the electrodes deposed by ink methods (e.g., screen printing) and/or plasma method. During formation, the respective electrodes, leads, heater(s), optional ground plane(s), optional temperature sensor(s), optional fugitive material(s), vias, and the like, are disposed onto the appropriate layers. The layers are laid-up and then fired at temperatures of about 1,400°C to about 1,500°C. Alternatively, the electrodes are not disposed onto the layers. The green layers (including the leads, optional ground plane(s), optional temperature sensor(s), optional fugitive material(s), vias, and the like) are fired at temperatures sufficient to sinter the layers, e.g., temperatures of about 1,400°C to about 1,500°C. The electrodes are then disposed on the appropriate fired layer(s), and the layers are laid-up accordingly. The sensor element is then again fired at a temperature sufficient to activate the electrode materials, e.g., temperatures of about 700°C to about 850°C.

With continued reference to Figure 1, a calibration resistor 94 is disposed on the top surface of layer 20. Electrical connection is made from the calibration resistor 94 to the contact pads 68a and 68b by way of leads 96. In such a way, one end of the calibration resistor 94 is electrically connected to contact pad 68a / lead 76 / electrode 34, and the other end of the calibration resistor 94 is electrically connected to contact pad 70b / lead 78 / electrode 36.

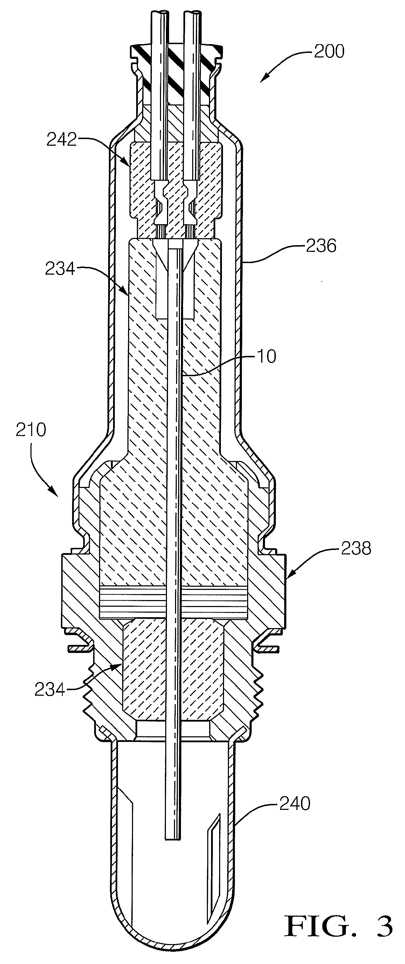

In one mode of use suited for sensing gas species in internal combustion engine exhaust gas, a sensor element such as sensor element 10 can be part of a gas sensor as shown in Figure 3. In gas sensor 200, sensor element 10 is mounted in a housing 210 by which the sensor element 10 can be secured to a conduit for an unknown gas, e.g., the exhaust pipe of an engine, and which permits the connection of a wiring harness to the sensor. In the embodiment of Figure 3, the housing comprises an insulator 234, an upper shell 236, a lower shell 238, and an outer shield 240. Sensor element 10 is disposed in an insulator 234, from which the sensing end of sensor element 10 protrudes and from which contact pads 68a, 68b, 68c, 70a, 70b, and 70c are accessible for connection with a wiring harness 242, which facilitates establishing electrical communication between sensor element 10 and a sensor circuit. Insulator 234 and sensor element 10 are protected in part by a lower shell 238, from which the sensing end of sensor element 10 protrudes, and an upper shell 236, which is mounted on lower shell 238 and through which the wiring harness 242 connected to contact pads 68a, 68b, 68c, 70a, 70b, and 70c can pass. Outer shield 240 is connected to lower shell 238 to protect the sensing end of sensor element 10, and is configured to permit a surrounding unknown gas to flow therethrough for contact with sensor element 10.

The foregoing description and associated Figures show that sensor element 10 comprises an ammonia-sensing cell, an A/F cell and a heater that are insulated from each other by insulating layers. Housing 210 is configured to permit gas flow therethrough for contact of the unknown gas with sensor element 10 and to permit sensor element 10, i.e., any one or more of A/F cell, ammonia-sensing cell and the heater of sensor element 10, to communicate with a device (e.g., a sensor circuit or control module) outside the housing.

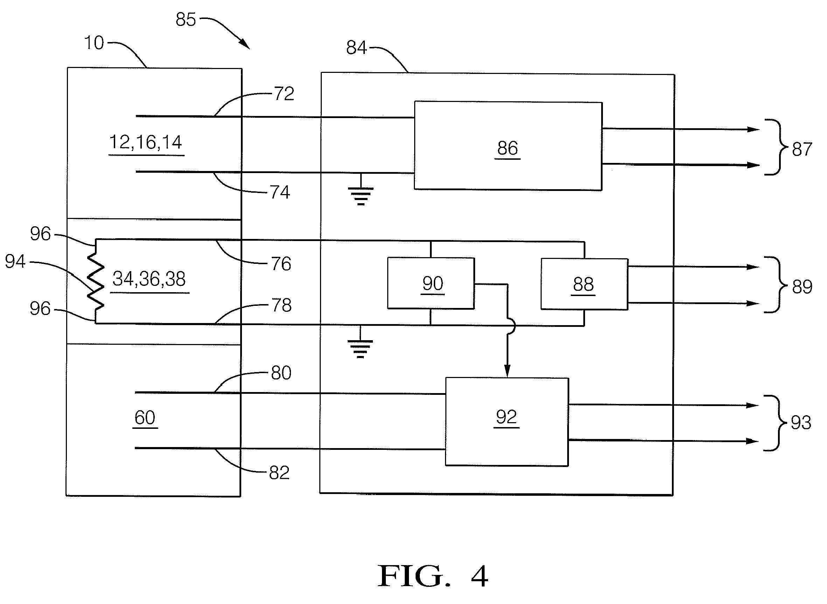

One embodiment of a sensor circuit 84 for use with sensor element 10 to form a sensor system 85 is shown schematically in Figure 4. Sensor circuit 84 is in electrical communication with leads 72, 74, 76, 78, 80, 82 and the electrodes and heater in communication therewith via contact pads 68a, 68b, 68c, 70a, 70b, and 70c (Fig. 1). Sensor circuit 84 includes an emf signal processing circuit ("emf processor") 86 in electrical communication with ammonia-sensing cell 12/16/14, and responsive thereto, for generating a signal indicating the content of the sensed species in the unknown gas (the "species gas output signal"), which can be emitted at output 87. Sensor circuit 84 also comprises a DC supply/sensor circuit 88 in electrical communication with A/F cell 34/38/36. DC supply/sensor circuit 88 is configured to provide a constant emf across the electrodes 34 and 36 and to sense the current through A/F cell 34/38/36 and generate a signal at output 89 that indicates the oxygen content or air-to-fuel ratio of the unknown gas. DC supply/sensor circuit 88 can be configured to receive and process, or even to generate, a rich/lean signal.

Sensor circuit 84 (Figure 4) can comprise an alternating voltage supply and sensing circuit (VAC supply/sensor circuit) 90 in electrical communication with one of the cells in the sensor element, sometimes referred to herein as a temperature cell. The application of an alternating voltage potential to cell electrodes on either side of an electrolyte material permits sensing of the resistivity (impedance) of the electrolyte material in the vicinity of the electrodes. The resistivity of an electrolyte layer is temperature-dependent, and VAC supply/sensor circuit 90 comprises processing circuitry for sensing the resistivity of the electrolyte layer and for providing a feedback signal to a heater control circuit 92 of sensor circuit 84. The heater control circuit 92 can be configured to adjust the power provided to heater 60 in response to the feedback signal to attain a selected working temperature for sensor element 10. Thus, heater control circuit 92 can be configured to operate in a feedback response mode to modulate the power provided to heater 60. In the illustrated embodiment, VAC supply/sensor circuit 90 communicates with a temperature cell comprising the A/F cell 34/38/36, via leads 76 and 78. VAC supply/sensor circuit 90 is also in electrical communication with a heater control circuit 92, which, in turn, is in electrical communication with heater 60 via leads 80 and 82. VAC supply/sensor circuit 90 is configured to apply an AC potential across A/F cell 34/38/36, from which the resistivity of the electrolyte and can be determined and a signal indicating the temperature of the cell can be generated.

Since the temperature of the sensor element in the vicinity of the electrodes is affected in significant part by the temperature of the gas to which the sensor element is exposed, the resistivity signal and/or the degree of power delivered to the heater by control circuit 92 can be processed as an indirect indication of the temperature of the unknown gas. In this way, the gas-sensing operation of the cell proceeds simultaneously with the operation of the AC sensing function of sensor circuit 84. In an alternative embodiment, the exhaust gas temperature could be measured directly by turning off heater 60, allowing sensor element 10 to reach thermal equilibrium with the exhaust gas, and then measuring the AC resistivity of layer 38.

Sensor circuit 84 can comprise a temperature signal output 93 for providing to other control circuits a signal indicating gas temperature. For example, a temperature signal could be provided to a controller for the engine producing unknown gas, so that engine performance can be adjusted in response to exhaust temperature.

In operation, heater control circuit 92 powers heater 60 to heat sensor element 10 to a working temperature, and sensor element 10 is exposed to an unknown gas. As a result, electrode 12 is disposed in fluid communication with the unknown gas via layer 18, and electrodes 14 and 34 are in fluid communication with the unknown gas via the aperture between layers 26 and 28. Similarly, electrode 36 is in diffusion-limited fluid communication with the unknown gas via material 52.

Sensor circuit 84 applies a voltage to A/F cell 34/38/36, causing oxygen to be pumped from electrode 36 to electrode 34 from where oxygen is emitted via aperture 30 and the open gas aperture 33 (Figure 2) between layers 28 and 26. The supply of gas to A/F cell 34/38/36 is limited by the diffusion-limiting channel from material 52, and the current through A/F cell 34/38/36 is sensed by DC supply/sensor circuit 88, which provides a quantitative indication of the oxygen content of the exhaust gas at output 89, from which the air/fuel ratio of the gas can be determined. Porous material 52 is sufficiently less porous than material 30 such that the oxygen level at electrode 14 will not much deviate from the oxygen concentration of the exposed gas. At the same time, VAC supply/sensor circuit 90 applies an alternating voltage (VAC) to electrodes 34 and 36. The VAC can have a frequency of about 1000 hertz (hz) to about 10 megahertz (Mhz) and an amplitude of about 10 millivolts (mv) to about 2000 mv. VAC supply/sensor circuit 90 senses the resistivity of the electrolyte layer 38 between the electrodes and provides a feedback signal to heater control circuit 92, which is responsive thereto. If the resistivity of layer 38 indicates that sensor element 10 is at a selected working temperature, power to heater 60 can be suspended; otherwise, power to heater 60 can be continued or increased as needed. Optionally, VAC supply/sensor circuit 90 can provide a temperature signal at output 93, for use by other control systems. Meanwhile, the exposure of electrodes 12 and 14 to an unknown gas containing ammonia results in an emf (i.e., voltage potential) between those electrodes 12 and 14 which can be processed by emf processor 86 to yield a quantitative indication of the ammonia content of the exhaust gas at output 87 based on the output signal from ammonia-sensing cell 12/16/14 and the oxygen and water content of the unknown gas, optionally determined from the A/F ratio derived from A/F cell 12/16/14. Hence, the NH₃ concentration, air/fuel ratio, and unknown gas temperature can all be determined from a single sensor. Sensor circuit 84 is configured to generate a signal indicating the NN₃ concentration (the ammonia concentration signal) of the unknown gas. Sensor circuit 84 may also generate a signal indicating the A/F ratio or oxygen content of the unknown gas.

The impedance vs. temperature characteristic of the electrolyte material 38 used in the temperature cell 34/38/36 is such that it appears essentially as an open circuit, i.e. having an impedance greater than 30 megohms, when the sensor element 10 is at ambient temperature. When the sensor 10 is at its operating temperature, the impedance of the temperature cell 34/38/36 decreases to a value in the range of 200 ohms to 600 ohms. The present invention takes advantage of this impedance vs. temperature characteristic by electrically connecting the calibration resistor 94 electrically in parallel with the temperature cell. With continued reference to Figure 4, the calibration resistor is shown in the sensor element 10 connected between lead 74 and lead 76 as indicated in the exploded view of Figure 1. Lead 74 is connected to ground in the sensor circuit 84, as is lead 78. As a result, when the sensing element 10 is connected to the sensor circuit 84 as shown in Figure 4, the calibration resistor 94 is connected electrically in parallel with the temperature cell 34/38/36.

In a preferred embodiment, the calibration resistor 94 is set to a resistance value at least 100 times the minimum expected impedance of the temperature cell 34/38/36 when the sensor element 10 is at its operating temperature. Under these conditions, the presence of the calibration resistor 94 in parallel with the temperature cell impedance will result a maximum impedance measurement error of 1% compared with measuring the temperature cell impedance on its own, i.e. without the calibration resistor 94 in parallel.

In an exemplary embodiment, the sensor element 10 is characterized during the manufacturing process for the sensor element 10, and the calibration resistor 94 is trimmed to a fixed value which is selected based on a measured performance characteristic of the sensor element 10. For example, a measurement may be made to characterize the output voltage of an ammonia sensor exposed to a predetermined concentration of ammonia gas, and the calibration resistor 94 trimmed to a resistance value having a predetermined relationship to the measured ammonia sensitivity of the sensor element 10. In a preferred embodiment, the calibration resistor 94 is a thick film resistor deposited on the sensor element 10, and laser trimming is used to adjust the resistance of the calibration resistor 94 to its target value. Other adjustable resistor means including but not limited to abrasive trimming and fusible links may be used in place of laser trimming without departing from the scope of the invention.

In an exemplary embodiment, the calibration resistor 94 is adjustable to a value in a range from a minimum of Rmin to a maximum of Rmax, where the ratio Rmax / Rmin is about 4. For example, for a temperature cell 34/38/36 having an impedance of 600 ohms at the sensor operating temperature, the calibration resistor 94 may be designed to have a minimum value Rmin of 60 kilohms (100 times the temperature cell impedance), and a maximum value Rmax of 240 kilohms (4 times Rmin). Having a resistance value for the calibration resistor 94 in this range ensures that the calibration resistor 94 in parallel with the temperature cell 34/38/36 introduces no more than 1% resistance error in the determination of the temperature cell impedance when the sensing element 10 is at its operating temperature.

In an exemplary method of using an exhaust sensor with a calibration resistor 94 electrically in parallel with a temperature cell, the resistance representing the parallel combination of the calibration resistor 94 and the temperature cell is measured at a time when the sensor element 100 is at a temperature below its operating temperature. For example, the resistance may be measured at vehicle key-on, before the heater 60 is energized to raise the sensor element 10 to its operating temperature. For the example presented above, with the calibration resistor 94 having a maximum value Rmax of 240 kilohms and the temperature cell having an impedance greater than 30 megohms at ambient temperature, the presence of the temperature cell in parallel with the calibration resistor 94 will affect the resistance measurement of the calibration resistor 94 by less than 1% compared to measuring the calibration resistor 94 on its own, i.e. without the temperature cell 94 in parallel.

In an alternative embodiment, the VAC can be applied to the ammonia-sensing cell 12/16/14 rather than A/F cell 34/38/36 to obtain the resistivity (impedance) feedback signal. In this alternative embodiment, the calibration resistor 94 would be disposed electrically in parallel with the sensing cell 12/16/14.

## Claims

1. An exhaust sensor (10) operable at an operating temperature greater than ambient temperature, said exhaust sensor (10) comprising:
a temperature cell (34/38/36) comprising an electrolyte material (38) in electrical contact with a first electrode (34) and a second electrode (36) and having a characteristic such that a temperature cell impedance measured between the first electrode (34) and the second electrode (36) varies as a function of the temperature of the electrolyte material (38), and
a resistor (94) having a fixed resistance value, said resistor (94) electrically connected between the first electrode (34) and the second electrode (36), the resistance value of the resistor (94) being intermediate between the temperature cell impedance when the exhaust sensor (10) is at ambient temperature and the temperature cell impedance when the exhaust sensor (10) is at the operating temperature.

2. The exhaust sensor (10) according to claim 1, wherein the fixed resistance value is selected as a function of a performance characteristic of the exhaust sensor (10).

3. The exhaust sensor (10) according to claim 2, wherein the performance characteristic of the exhaust sensor (10) is a sensitivity of the exhaust sensor (10) to a concentration of a gas species.

4. The exhaust sensor (10) according to claim 2, wherein the resistor (94) is formed as a film resistor deposited on a substrate (20).

5. The exhaust sensor (10) according to claim 4, wherein the resistor (94) is trimmed to the fixed resistance value using laser trimming.

6. A method of producing an exhaust sensor (10), said method comprising the steps of
disposing a first electrode (34) and a second electrode (36) in contact with an electrolyte (38) to form a temperature cell (34/38/36);
disposing an resistor (94) electrically connected to the first electrode (34) and the second electrode (36);
determining a performance characteristic of the exhaust sensor (10); and
adjusting the resistance value of the resistor (94) based on the determined performance characteristic of the exhaust sensor (10).

7. The method according to claim 6, wherein the performance characteristic of the exhaust sensor (10) is a sensitivity of the exhaust sensor (10) to a concentration of a gas species.

8. A method of using an exhaust sensor (10), said exhaust sensor (10) comprising a fixed resistor (94) electrically in parallel with a temperature cell (34/38/36) whose impedance varies as a function of the temperature of the exhaust sensor (10), the method comprising the steps of:
disposing the exhaust sensor (10) in an exhaust system so as to be exposed to exhaust gas;
measuring the impedance of the parallel combination of the fixed resistor (94) and the temperature cell (34/38/36) when the exhaust sensor (10) is at ambient temperature;
providing the measured ambient temperature impedance value to a controller configured to use the ambient temperature impedance value to determine a sensor compensation characteristic;
receiving a concentration signal from the exhaust sensor (10), said concentration signal being a function of a gas species concentration sensed by the exhaust sensor (10); and
adjusting the concentration signal based on the sensor compensation characteristic to determine a compensated gas species concentration.

9. The method according to claim 8, wherein the exhaust sensor (10) is disposed in a vehicle and wherein the step of measuring the impedance of the parallel combination of the fixed resistor (94) and the temperature cell (34/38/36) is performed at a key-on event of the vehicle.
